# EUROPEAN PATENT SPECIFICATION

(11) **EP 2 331 079 B1**
(45) Date of publication and mention of the grant of the patent: **18.12.2013**
(21) Application number: 09788682.4
(22) Date of filing: 24.08.2009
(51) Int. Cl.: A61K 9/20, A61K 31/4545, A61K 31/47, A61K 31/45

(54) **PHARMACEUTICAL FORMULATION**
PHARMAZEUTISCHE FORMULIERUNG
PRÉPARATION PHARMACEUTIQUE

(30) Priority: 22.08.2008 TR 200806298
(43) Date of publication of application: 15.06.2011
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2009/000107
(87) International publication number: WO 2010/021607

(56) References cited:
- WO-A1-97/28797
- WO-A2-03/101434
- WO-A2-2008/079312
- US-A1- 2007 224 266
- DAVIS ET AL: "Effect of combined montelukast and desloratadine on the early asthmatic response to inhaled allergen" JOURNAL OF ALLERGY AND CLINICAL IMMUNOLOGY, MOSBY, INC, US LNKD- DOI:10.1016/J.JACI.2005.06.014, vol. 116, no. 4, 1 October 2005 (2005-10-01), pages 768-772, XP005094467 ISSN: 0091-6749

## Description

### Field of the invention

The present invention relates to the pharmaceutical formulations comprising two active substances, and the methods for the preparation thereof, and the use thereof.

### Background of the invention

The present invention provides a combination effective in the prevention and/or treatment of allergic and inflammatory diseases of the skin or upper and lower airways such as seasonal allergic rhinitis, perennial allergic rhinitis, allergic sinusitis, atopic dermatitis, urticaria, allergic asthma, aspirin-induced asthma, exercise-induced asthma and concomitant symptoms associated therewith. This effect which is provided by the combination according to the present invention is hereinafter referred as "the desirable effect". The mentioned combination comprises desloratadine (or a pharmaceutically acceptable salt, solvate, derivative, polymorph, hydrate or enantiomer thereof) as a long-acting, non-sedating H1-antihistaminic and montelukast (or a pharmaceutically acceptable salt thereof) as a leukotriene receptor antagonist.

Allergic reactions (hypersensitivity reactions) are hypersensitive responses of the immune system to a harmless substance.

The immune system including antibodies, white blood cells, mast cells, complement proteins, and other substances defence the body against foreign substances called antigens. But, the immune system can overreact to certain antigens, called allergens, which are harmless in most people. The result is an allergic reaction.

When the allergens contact with the skin or eyes, or are inhaled, or are eaten or drunk, or are injected, they may cause an allergic reaction. An allergic reaction may also occur as a part of seasonal allergy as a result of periodic exposure.

On initial exposure to an allergen, the immune system produces the antibodies called immunoglobulin E (IgE). Secreted IgE circulates in the blood and binds to the white blood cells called tissue mast cells and blood basophils. Initial exposure to an allergen may cause sensitivity, but generally any symptom does not occur. If later exposure to the same allergen occurs, the cells of which the IgE molecules held on the surface release inflammatory chemical mediators such as histamine and leukotriene into the surrounding tissue causing inflammation and edema. These mediators initiate a series of reactions resulting in tissue damage.

Most of the allergic reactions are characterized by the symptoms of redness, itching and tearing of eyes (allergic conjunctivitis); nasal congestion, itching and running nose (allergic rhinitis); allergic sinusitis; skin rashes, itching and redness of the skin (urticaria); abdominal pain, bloating, vomiting, diarrhea in the gastrointestinal system; feeling of fullness, possibly pain and impaired hearing due to the lack of eustachian tube drainage in the ears; sneezing, coughing, bronchoconstriction, wheezing, dyspnea, sometimes outright attacks of asthma and in severe cases airway constriction due to swelling known as angioedema in the airways.

Allergies may also trigger the asthma attacks. Asthma, which comes as attacks, is a reversible airway obstruction characterized by dyspnea and wheezing. Asthma is one of the most common chronic diseases in children and adults. The reasons for the increase in the asthma prevalence are not known exactly. But it is thought that genetic factors, allergens, some foods, some drugs (aspirin), respiratory diseases, exercise, smoke, dominant odor and sprays, chemicals, industrialization, air pollution and change of air are effective in increasing the asthma prevalence.

Airways are flexible structures that can change its diameter as a response to different stimulus. But, airways are hypersensitive to non-specific stimulus in the case of asthma. The sensitized airways exhibit excessive bronchoconstrictor response even if exposed to simple stimulus that does not affect the healthy people.

Obstruction of the airways is often caused by abnormal sensitivity of cholinergic receptors, which cause the muscles of the airways to contract when they should not. Certain cells in the airways, particularly mast cells, are thought to be responsible for initiating the response. Mast cells release inflammatory chemical mediators such as histamine and leukotriene that cause smooth muscle contraction, increase in mucus secretion and migration of certain white blood cells to the inflammation area. Eosinophils are also thought to contribute to airway obstruction.

Characteristic symptoms of the asthma are expectoration, coughing, wheezing, dyspnea and chest tightness. All of these symptoms may not occur at the same time. Symptoms of asthma vary in frequency and severity patient by patient and within the time in the same patient.

It is determined in the studies that 38% of the patients with allergic rhinitis have also asthma, and 78% of the patients with asthma have also allergic rhinitis. Bronchial hyperreactivity is found to be high in the respiratory function tests applied to the patients with allergic rhinitis although they do not have symptoms of asthma. It is believed that the risk of developing asthma in patients with rhinitis is three times more than the risk of developing asthma in patients without rhinitis and rhinitis is a risk factor for asthma. Untreated allergic rhinitis in patients with asthma which allergic rhinitis accompanies is found to affect asthma management in a negative way.

Many studies show that the treatment of allergic rhinitis in patients with asthma results in improvement of the asthma symptoms, decrease in bronchial sensitivity, protection against bronchospasm and decrease in the use of rescue medicines.

The similarity of the immunopathological processes such as allergic hypersensitivity responses and persistent allergic inflammation underlying allergic rhinitis and asthma requires similar treatment regimens. The objective of the treatment in both of the diseases is to manage the inflammation of the airways.

In that case according to the invention, combination of a potent antihistaminic and a potent leukotriene receptor antagonist is preferred as an anti-inflammatory treatment so as to prevent chemical mediators such as histamine and leukotriene to initiate the inflammative reactions.

In addition, treatment in patients with chronic urticaria, which is another allergy-induced disease, alone or accompanied asthma is difficult and urticaria may not be managed with only antihistaminics. Because, the chemical mediators including quinines, prostaglandins and leukotrienes excluding histamine may be responsible for some symptoms of urticaria which can not be managed with antihistaminics. In that case according to the invention, leukotriene antagonists must be used in addition to the antihistaminics in the treatment of patients with chronic urticaria for optimum efficacy.

Antihistaminics are defined by the histamine receptors which they interact with. H1-antihistaminics are commonly used for the allergic disorders. H1-antihistaminics are fast-acting drugs, and their effect is long-lasting (at least 24 hours) and reproducible. H1-antihistaminics exhibit very little side effects, particularly on central nervous system, and do not interact with other drugs.

The present invention provides a combination of an H1-antihistaminic and a leukotriene receptor antagonist effective in the prevention and/or treatment of allergic and inflammatory diseases of the skin or upper and lower airways and concomitant symptoms associated therewith.

Desloratadine is a long-acting, non-sedating H1-antihistaminic with a chemical name of 8-chloro -6,11-dihydro -11- (4-piperidinylidene) -5H- benzo [5,6] cyclohepta [1,2-b] pyridine (Formula I).

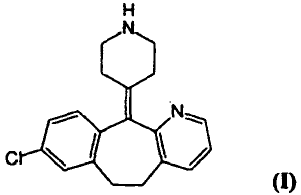

Desloratadine is disclosed for the first time in the patent numbered US4659716 A (EP0152897 B1, EP0208855 B1, US4731447 A, US4873335 A, WO8503707 A1 are in the same patent family). Processes for preparing desloratadine, pharmaceutical compositions comprising desloratadine and the use of desloratadine as a non-sedating antihistaminic are also disclosed.

Desloratadine, an active metabolite of loratadine, is an oral H1-receptor antagonist. Desloratadine is a new antihistaminic which binds to the H1 receptors with a higher affinity than the other H1-receptor antagonists. Desloratadine competes with histamine and inhibits the binding of histamine to the H1 receptors. This antagonism blocks the effect of histamine on the gastrointestinal tract, uterus, dilated blood vessels and bronchial smooth muscle. The blockage of the H1 receptors also inhibits the histaminic activities such as edema, flare and itching. The non-sedating property of the H1-receptor antagonists is partly explained by passing into central nervous system in low amounts and the low affinity to the H1 receptors therein.

Desloratadine has an anti-allergic and anti-inflammatory activity. The studies show that desloratadine inhibits a comprehensive series of reactions that initiates and generalizes allergic inflammation.

Montelukast is a leukotriene receptor antagonist with a chemical name of 1-[[[(R)-1-[3-[(1E)-2-(7-chloro-2-quinolinyl)ethenyl] phenyl] -3- [2-(1-hydroxy-1-methyl-ethyl)phenyl] propyl] thio] methyl] cyclopropane acetic acid (Formula II).

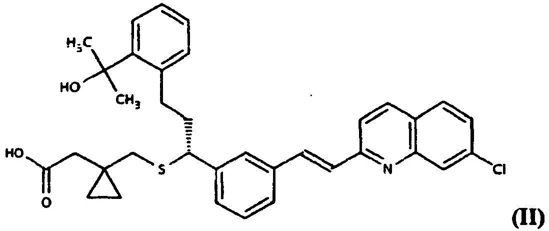

Montelukast is disclosed for the first time in the patent numbered EP480717 B1 (US5565473 A and US5856322 A are in the same patent family). Processes for preparing montelukast and the use of montelukast as a leukotriene antagonist are also disclosed.

Montelukast is an orally active potent and selective antagonist of leukotriene D4 (LTD₄) which has activities at the cysteinyl leukotriene receptor (CysLT₁) found in the airways. Cysteinyl leukotrienes (LTC₄, LTD₄, LTE₄) are potent inflammatory eicosanoids released from various cells including mast cells and eosinophils. These pro-asthmatic mediators bind to the cysteinyl leukotriene receptors (CysLT) found in the airways and cause a series of respiratory activities such as bronchoconstriction, viscous mucus secretion, vascular permeability and eosinophil accumulation.

Montelukast is a potent compound which improves asthma inflammation parameters significantly. Montelukast binds with high affinity and selectivity to the CysLT₁ receptor and inhibits physiologic actions of LTC₄, LTD₄, LTE₄ at the CysLT₁ receptor without any agonist activity.

Synergistically increasing anti-inflammatory effect of desloratadine and montelukast was proven by the various clinical trials:
- Maciej C.; Malgorzata G-C.; Lawrence M.D.; Pawel G. Montelukast with desloratadine or levocetirizine for the treatment of persistent allergic rhinitis. Annals of allergy, asthma & immunology 2006, 97(5), pp. 664-671
- Ciebiada M ., Górska-Ciebiada M ., DuBuske L ., Gorski P. Combination Therapy with Montelukast and Antihistamines (desloratadine or Levocetirizine) Improves Quality of Life in Patients with Persistent Allergic Rhinitis: a Double Blind Placebo Controlled Study. Journal of Allergy and Clinical Immunology, 117(2), p.165
- Nettis E., Colanardi M.C., Paradiso M.T. ve Ferrannini A. Desloratadine in combination with montelukast in the treatment of chronic urticaria: a randomized, double-blind, placebo-controlled study. Clin. Exp. Allergy 2004; 34: 1401-1407
- Nettis, E.; Colanardi, M:C.; Soccio, A.L.; Ferrannini, A.; Vacca, A. Desloratadine in combination with montelukast suppresses the dermographometer challenge test papule, and is effective in the treatment of delayed pressure urticaria: a randomized, double-blind, placebo-controlled study. British Journal of Dermatology 2006, 155(6), pp. 1279-1282
- Davis B. E., BSc, Todd D. C., MD, FRCP(C), Cockcroft D. W., MD, FRCP(C). Effect of combined montelukast and desloratadine on the early asthmatic response to inhaled allergen. Journal of Allergy and Clinical Immunology 2005, 116(4), pp. 768-772

The present invention is directed to obtain a combination of desloratadine (or a pharmaceutically acceptable salt, solvate, derivative, polymorph, hydrate or enantiomer thereof) and montelukast (or a pharmaceutically acceptable salt thereof), which provides the desirable effect, depending on synergistically increasing anti-inflammatory effect of desloratadine and montelukast.

The object of the invention is to provide a dosage form such as tablet by combining pharmaceutically acceptable, non-toxic and therapeutically effective amount of desloratadine (or a pharmaceutically acceptable salt, solvate, derivative, polymorph, hydrate or enantiomer thereof) and montelukast (or a pharmaceutically acceptable salt thereof) in a manner so as to obtain the desirable effect.

But the studies show that the pharmaceutical composition comprising desloratadine and montelukast as active substances has a stability problem resulting from both of the substances, and accordingly incompatibility problem between the components of the composition.

Pharmaceutical compositions comprising desloratadine are prone to degrade chemically and physically due to temperature and pressure conditions. In addition, because desloratadine is soluble in an acidic pH, the use of an acidic excipient causes degradation in the structure and color of desloratadine. The excipients incompatible with desloratadine are the substances such as stearic acid, povidone, crospovidone, lactose, lactose monohydrate, sodium benzoate and gliceryl behenate. This condition brings about the need of choosing the suitable excipient composition to provide the stabilization of desloratadine in the tablet.

Stable pharmaceutical compositions for oral administration comprising an anti-allergic effective amount of desloratadine in a pharmaceutically acceptable carrier medium comprising a desloratadine-protective amount of a pharmaceutically acceptable basic salt and at least one pharmaceutically acceptable disintegrant, are disclosed in the patent numbered US6100274 A.

Stable pharmaceutical compositions comprising an anti-allergic effective amount of desloratadine in a pharmaceutically acceptable carrier medium comprising a pharmaceutically acceptable basic salt, wherein the pharmaceutically acceptable carrier medium is substantially free of acidic excipients, and wherein the pharmaceutically acceptable basic salt is a carbonate, phosphate, silicate or sulfate of calcium, magnesium or aluminium or mixtures thereof, are disclosed in the patent numbered EP1073438 BI.

Reactivity between lactose and desloratadine, and stable, non-hygroscopic, anhydrous pharmaceutical compositions free of mono- and di-saccharides, are disclosed in the patent numbered EP0969836 B 1.

Stable pharmaceutical compositions comprising a therapeutically effective amount of desloratadine, a stabilizer selected from the group comprising an antioxidant, a pharmaceutically acceptable organic compound that provides an alkaline pH, an alkali metal salt, or mixtures thereof, and pharmaceutically acceptable excipients, are disclosed in the patent application numbered WO2005065047 A2.

Stable pharmaceutical compositions of desloratadine comprising desloratadine in combination with a desloratadine-stabilizing amount of at least one amino acid, are disclosed in the patent application numbered EP1728513 A2.

Stable pharmaceutical compositions comprising desloratadine or a pharmaceutically acceptable salt, solvate, derivative, polymorph, hydrate or enantiomer thereof, and a carrier comprising at least one polyol, are disclosed in the patent application numbered WO2006008512 A2.

As for montelukast, montelukast is highly hygroscopic and very sensitive to light and oxygen. So, pharmaceutical compositions comprising montelukast are prone to degradation due to the production and storage conditions. This condition brings about the need of choosing the suitable excipient composition to provide the stabilization of montelukast in the tablet.

Stable pharmaceutical compositions comprising montelukast or a salt thereof and a pharmaceutically acceptable excipient selected from at least one of diluent, binder, disintegrant, wetting agent, lubricant, or glidant, provided that the pharmaceutically acceptable excipient is not microcrystalline cellulose, are disclosed in the patent application numbered EP1818057 A1.

The solutions recommended in the above-mentioned patents and patent applications are directed to pharmaceutical compositions comprising desloratadine alone or montelukast alone. When desloratadine and montelukast are combined in a single dosage form, problems resulting from formulating the active substances together in addition to the stability problems of each active substance also arise. The use of the excipients such as stearic acid, povidone, crospovidone and lactose required for formulating montelukast causes degradation of desloratadine in the pharmaceutical formulations comprising montelukast in combination with desloratadine. So, incompatibility problem arises between the components of the composition while preparing a pharmaceutical formulation comprising both desloratadine and montelukast. Pharmaceutical compositions comprising an effective amount of at least one leukotriene antagonist selected from montelukast or pranlukast or a pharmaceutically acceptable salt thereof in admixture with an effective amount of at least one antihistamine which is descarboethoxyloratidine, fexofenadine, ebastine, norastemizole or a pharmaceutically acceptable salt thereof, are disclosed in the patent numbered EP1041990 B1. It is defined in the patent depending on the therapeutical activities of montelukast alone and desloratadine alone that montelukast and desloratadine may be combined, but there is not any suggestion for formulation presented in the patent. Additionally, the main problem of incompatibility between the components of the composition is not mentioned in the patent, accordingly there is not any solution for this problem presented in the patent.

The present invention provides a composition and a manufacturing method that solve both the stability problems of each active substance and incompatibility problem between the components of the composition so as to obtain the desirable effect.

### Summary of the invention

The present invention relates to a process for the preparation of a pharmaceutical composition comprising therapeutically effective amount of desloratadine or a pharmaceutically acceptable salt, solvate, derivative, polymorph, hydrate or enantiomer thereof and therapeutically effective amount of montelukast or a pharmaceutically acceptable salt thereof for use in the manufacture of a medicament for the prevention and/or treatment of allergic and inflammatory diseases of the skin or upper and lower airways such as seasonal allergic rhinitis, perennial allergic rhinitis, allergic sinusitis, atopic dermatitis, urticaria, allergic asthma, aspirin-induced asthma, exercise-induced asthma and concomitant symptoms associated therewith, characterized in that desloratadine or a pharmaceutically acceptable salt, solvate, derivative, polymorph, hydrate or enantiomer thereof and montelukast or a pharmaceutically acceptable salt thereof are preformulated in a series of manufacturing process steps.

According to the present invention, the manufacturing process which provides a formulation so as to obtain the desirable effect is as follows:
- desloratadine or a pharmaceutically acceptable salt, solvate, derivative, polymorph, hydrate or enantiomer thereof and at least one pharmaceutically acceptable stabilizing agent and optionally other pharmaceutically acceptable excipients are mixed to obtain the first mixture;
- montelukast or a pharmaceutically acceptable salt thereof and at least one pharmaceutically acceptable stabilizing agent and optionally other pharmaceutically acceptable excipients are mixed to obtain the second mixture;
- both of the mixtures are fed separately to the tablet press machine;
- tablets obtained in the previous step are optionally film-coated.

### Detailed description of the invention

The present invention is directed to obtain a combination of desloratadine (or a pharmaceutically acceptable salt, solvate, derivative, polymorph, hydrate or enantiomer thereof) and montelukast (or a pharmaceutically acceptable salt thereof), which provides the desirable effect, depending on synergistically increasing anti-inflammatory effect of desloratadine and montelukast. But, as defined before, there are stability problems of each active substance and incompatibility problem between the components of the composition in a combined preparation which are faced during the studies. To make sure the combination of desloratadine and montelukast provides the desirable effect, on the one hand stability problem of the active substances must be overcome, and on the other hand therapeutically effective amounts of the active components and the suitable composition of the excipients must be found, and on the another hand the active substances must be formulated separately.

Surprisingly, it is found that a pharmaceutical composition comprising desloratadine or a pharmaceutically acceptable salt, solvate, derivative, polymorph, hydrate or enantiomer thereof and montelukast or a pharmaceutically acceptable salt thereof, wherein desloratadine or a pharmaceutically acceptable salt, solvate, derivative, polymorph, hydrate or enantiomer thereof and montelukast or a pharmaceutically acceptable salt thereof are preformulated, has an optimum efficacy in the prevention and/or treatment of allergic and inflammatory diseases of the skin or upper and lower airways and concomitant symptoms associated therewith.

According to another aspect of the invention, a pharmaceutical composition comprising desloratadine or a pharmaceutically acceptable salt, solvate, derivative, polymorph, hydrate or enantiomer thereof in a specific amount, montelukast or a pharmaceutically acceptable salt thereof in a specific amount, at least one pharmaceutically acceptable stabilizing agent for each active substance in an adequate amount and optionally one or more pharmaceutically acceptable excipients selected from the additives such as diluents, binders, disintegrants, lubricants, glidants and surfactants, has an optimum efficacy in the prevention and/or treatment of allergic and inflammatory diseases of the skin or upper and lower airways and concomitant symptoms associated therewith.

Stability problem of the active substances is solved by applying a series of manufacturing process steps to the active substances due to their degradation properties; and incompatibility problem between the components of the composition is solved by formulating the active substances separately and not mixing them together.
- In the first step, the first mixture is obtained by mixing desloratadine or a pharmaceutically acceptable salt, solvate, derivative, polymorph, hydrate or enantiomer thereof and at least one pharmaceutically acceptable stabilizing agent and optionally other pharmaceutically acceptable excipients together.
- In the second step, the second mixture is obtained by mixing montelukast or a pharmaceutically acceptable salt thereof and at least one pharmaceutically acceptable stabilizing agent and optionally other pharmaceutically acceptable excipients together.
- Both of the mixtures are fed separately to the tablet press machine.
- Finally, tablets obtained in the previous step are optionally film-coated.

The term "allergic and inflammatory diseases" as used herein refers to allergic and inflammatory diseases of the skin or upper and lower airways such as seasonal allergic rhinitis, perennial allergic rhinitis, allergic sinusitis, atopic dermatitis, urticaria, allergic asthma, aspirin-induced asthma, exercise-induced asthma.

The term "concomitant symptoms associated therewith" as used herein refers to redness, itching and tearing of eyes, nasal congestion, itching and running nose, sneezing, coughing, skin rashes, itching and redness of the skin, bronchoconstriction, wheezing, dyspnea, airway constriction due to swelling, expectoration and attacks of asthma.

The terms "in a specific amount", "in an adequate amount" and "optionally" as used herein refer to desloratadine or a pharmaceutically acceptable salt, solvate, derivative, polymorph, hydrate or enantiomer thereof in an amount of from about 0.1 to 20% by weight, montelukast or a pharmaceutically acceptable salt thereof in an amount of from about 0.1 to 20% by weight, at least one pharmaceutically acceptable stabilizing agent for each active substance in an amount up to about 10% by weight and one or more pharmaceutically acceptable excipients selected from the additives such as diluents, binders, disintegrants, lubricants, glidants and surfactants which are used when needed, which all of them are preferred to obtain the desirable effect.

"Pharmaceutically acceptable salt of montelukast" may be derived from inorganic bases selected from alkali metals such as sodium, lithium and potassium, alkaline earth metals such as calcium and magnesium, and aluminium, zinc, ammonium, and the like; or organic bases selected from arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, isopropylamine, lysine, methylglucosamine, morpholine, piperazine, piperidine, polyamine resins, procaine, purine, theobromine, triethylamine, trimethylamine, basic ion-exchange resins such as tripropylamine, cyclic amines, substituted amines, primary, secondary, tertiary amines, and the like; and is preferably sodium salt.

Pharmaceutically acceptable stabilizing agents may be selected from antioxidants, chelating agents, alkalinizing agents, photoprotectants, and the like.

Pharmaceutically acceptable antioxidants may be selected from butylated hydroxyanisole (BHA), sodium ascorbate, butylated hydroxytoluene (BHT), sodium sulfite, gallates (such as propyl gallate), tocopherol, citric acid, malic acid, ascorbic acid, acetylcysteine, fumaric acid, lecithine, ascorbyl palmitate, ethylenediamine tetraacetate, and the like.

Pharmaceutically acceptable chelating agents may be selected from disodium EDTA, edetic acid, citric acid, sodium citrate, potassium citrate or combinations thereof, and the like. These agents prevent the oxidation by surrounding the metal ions that may catalyse the oxidation process.

Pharmaceutically acceptable alkalinizing agents may be selected from alkali metal salts such as sodium carbonate, sodium hydroxide, sodium silicate, disodium hydrogen orthophosphate, sodium aluminate, and the like; alkaline earth metal salts such as calcium carbonate, calcium hydroxide, dibasic calcium phosphate, tribasic calcium phosphate, calcium sulfate, calcium acetate, calcium gluconate, calcium glycerophosphate, magnesium carbonate, magnesium hydroxide, magnesium sulfate, magnesium acetate, magnesium silicate, magnesium aluminate, and the like; and organic compounds such as primary, secondary, tertiary amines, cyclic amines, N,N'-dibenzylethylenediamine, diethanolamine, ethylenediamine, meglumine, monosodium glutamate, polacrilline sodium, sodium alginate, and the like.

Pharmaceutically acceptable photoprotectants may be selected from metal oxides such as titanium oxide, ferric oxide, zinc oxide, and the like.

Pharmaceutically acceptable diluents may be selected from lactose, microcrystalline cellulose, starch, pregelatinized starch, modified starch, calcium phosphate (dibasic and/or tribasic), calcium sulfate trihydrate, calcium sulfate dihydrate, calcium carbonate, kaolin, lactilol, powder cellulose, dextrose, dextrates, dextrin, sucrose, maltose, fructose, mannitol, sorbitol, xylitol, and the like. Diluent is present in an amount within the range of preferably 0 to 95% by weight, more preferably 5 to 85% by weight.

Pharmaceutically acceptable binders may be selected from starches (such as potato starch, corn starch, wheat starch), sugars such as sucrose, glucose, dextrose, lactose and maltodextrin, natural and synthetic gums (such as acacia), gelatin, cellulose derivatives (such as microcrystalline cellulose, HPC, HEC, HPMC, carboxymethylcellulose, methylcellulose, ethylcellulose), polyvinylpyrrolidone, polyethylene glycol, waxes, calcium carbonate, calcium phosphate, alcohols (such as sorbitol, xylitol, mannitol), water, and the like. Binder is present in an amount within the range of preferably 0 to 10% by weight, more preferably 0.1 to 5% by weight.

Pharmaceutically acceptable disintegrants may be selected from starch (such as potato starch, corn starch), sodium starch glycolate, pregelatinized starch, cellulose derivatives (such as croscarmellose sodium, microcrystalline cellulose), polyvinylpyrrolidone, crospovidone, alginic acid, sodium alginate, clays (such as xanthan gum or Veegum), ion-exchange resins, effervescent systems such as those utilizing food acids and alkaline carbonate components, and the like. Preferably croscarmellose sodium and starch are used. Disintegrant is present in an amount within the range of preferably 0 to 10% by weight, more preferably 0.1 to 5% by weight.

Pharmaceutically acceptable lubricants may be selected from metallic stearates (such as magnesium stearate, calcium stearate, aluminium stearate), fatty acid esters (such as sodium stearyl fumarate), fatty acids (such as stearic acid), fatty alcohols, glyceryl behenate, mineral oils, paraffins, hydrogenated vegetable oils, leucine, polyethylene glycols, metallic lauryl sulfates (such as sodium lauryl sulfate, magnesium lauryl sulfate), sodium chloride, sodium benzoate, sodium acetate, talk, and the like. Preferably magnesium stearate is used. Lubricant is present in an amount within the range of preferably 0 to 10% by weight, more preferably 0.1 to 5% by weight.

Pharmaceutically acceptable glidants may be selected from silicon dioxide, magnesium trisilicate, powder cellulose, starch, talk, tribasic calcium phosphate, metallic stearates, calcium silicate, metallic lauryl sulfates, and the like. Glidant is present in an amount up to 1% by weight.

Pharmaceutically acceptable surfactants may be selected from polyoxyethylene-sorbitan-fatty acid esters (polysorbates), sodium lauryl sulfate, sodium stearyl fumarate, polyoxyethylene alkyl ethers, sorbitan fatty acid esters, polyethylene glycols, polyoxyethylene castor oil derivatives, docusate sodium, quaternary ammonium compounds, aminoacids such as L-leucine, sugar esters of fatty acids, glycerides of fatty acids, and the like. Surfactant is present in an amount within the range of preferably 0 to 10% by weight, more preferably 0.1 to 5% by weight.

In addition, solubility modulators, electrolytes, sweeteners, colorants, coating agents, and the like may be used in the formulation.

The examples according to the invention are given below. These examples are given to explain the invention, but does not limit the scope of the invention.

### Examples

### Example 1.

| **Ingredients** | **Quantity (mg)** |
|---|---|
| **Montelukast sodium *** | 10.4 |
| Mannitol | 140.87 |
| Red iron oxide | 0.03 |
| Povidone | 4 |
| Crospovidone | 6 |
| HPC | 7 |
| Colloidal silicon dioxide | 0.3 |
| L-leucine | 10 |
| **Desloratadine** | 5 |
| Calcium hydrogen phosphate dihydrate | 38 |
| Microcrystalline cellulose | 50.2 |
| Corn starch | 8 |
| Colloidal silicon dioxide | 0.3 |
| Talk | 3 |
| Citric acid anhydrous | 1.5 |
| Sodium stearyl fumarate | 0.4 |
| Total weight | 285 |
| **Coating material** | 5 |

| | |
|---|---|
| * Equivalent to 10 mg of montelukast | |

### Example 2.

| **Ingredients** | **Quantity (mg)** |
|---|---|
| **Montelukast sodium *** | 10.4 |
| Mannitol | 153.16 |
| Red iron oxide | 0.04 |
| Povidone | 4.2 |
| Crospovidone | 5.6 |
| Magnesium stearate | 2 |
| **Desloratadine** | 5 |
| Calcium hydrogen phosphate dihydrate | 34 |
| Microcrystalline cellulose | 54.5 |
| Starch | 5 |
| Talk | 4 |
| Citric acid anhydrous | 2 |
| BHA | 0.1 |
| Total weight | 280 |
| **Coating material** | 5 |

| | |
|---|---|
| * Equivalent to 10 mg of montelukast | |

## Claims

1. A process for the preparation of a pharmaceutical tablet composition comprising therapeutically effective amount of desloratadine or a pharmaceutically acceptable salt, solvate, polymorph or hydrate thereof and therapeutically effective amount of montelukast or a pharmaceutically acceptable salt **characterized in that** desloratadine or a pharmaceutically acceptable salt, solvate, polymorph or hydrate thereof and montelukast or a pharmaceutically acceptable salt thereof are preformulated in a series of manufacturing process steps comprising;
a. preparing the preformulation of desloratadine by mixing desloratadine or a pharmaceutically acceptable salt, solvate, polymorph or hydrate thereof and at least one pharmaceutically acceptable stabilizing agent selected from the group consisting of antioxidants, chelating agents, alkalinizing agents and photoprotectants; and optionally other pharmaceutically acceptable excipients,
b. preparing the preformulation of montelukast by mixing montelukast or a pharmaceutically acceptable salt thereof and at least one pharmaceutically acceptable stabilizing agent selected from the group consisting of antioxidants, chelating agents, alkalinizing agents and photoprotectants; and optionally other pharmaceutically acceptable excipients,
c. feeding the preformulations obtained in previous steps separately to the tablet press machine,
d. optionally, coating the tablets obtained in the step c).

2. A pharmaceutical tablet composition prepared according to claim 1.

3. The pharmaceutical tablet composition prepared according to claim 2, **characterized in that** the pharmaceutical tablet composition comprises, based on the weight of the core tablet,
- desloratadine or a pharmaceutically acceptable salt, solvate, derivative, polymorph, hydrate or enantiomer thereof in an amount of from about 0.1 to 20% by weight,
- montelukast or a pharmaceutically acceptable salt thereof in an amount of from about 0.1 to 20% by weight,
- at least one pharmaceutically acceptable stabilizing agent for each active substance in an amount up to about 10% by weight, and
- optionally one or more pharmaceutically acceptable excipients selected from the additives such as diluents, binders, disintegrants, lubricants, glidants and surfactants.

4. The pharmaceutical tablet composition according to claim 2 for use in the treatment of allergic and inflammatory diseases of the skin or upper and lower airways such as seasonal allergic rhinitis, perennial allergic rhinitis, allergic sinusitis, atopic dermatitis, urticaria, allergic asthma, aspirin-induced asthma, exercise-induced asthma.

5. The pharmaceutical tablet composition prepared according to claim 2 **characterized in that** the pharmaceutical tablet composition comprises sodium salt of montelukast as a pharmaceutically acceptable salt of montelukast.

6. The pharmaceutical tablet composition prepared according to claim 2 **characterized in that** the pharmaceutical tablet composition comprises an antioxidant as a stabilizing agent selected from butylated hydroxyanisole (BHA), sodium ascorbate, butylated hydroxytoluene (BHT), sodium sulfite, gallates, tocopherol, citric acid, malic acid, ascorbic acid, acetylcysteine, fumaric acid, lecithine, ascorbyl palmitate and ethylenediamine tetraacetate.

7. The pharmaceutical tablet composition prepared according claim 2 **characterized in that** the pharmaceutical tablet composition comprises a chelating agent as a stabilizing agent selected from disodium EDTA, edetic acid, citric acid, sodium citrate, potassium citrate or combinations thereof.

8. The pharmaceutical tablet composition prepared according to claim 2 **characterized in that** the pharmaceutical tablet composition comprises an alkalinizing agent as a stabilizing agent selected from alkali metal salts including sodium carbonate, sodium hydroxide, sodium silicate, disodium hydrogen orthophosphate and sodium aluminate; alkaline earth metal salts including calcium carbonate, calcium hydroxide, dibasic calcium phosphate, tribasic calcium phosphate, calcium sulfate, calcium acetate, calcium gluconate, calcium glycerophosphate, magnesium carbonate, magnesium hydroxide, magnesium sulfate, magnesium acetate, magnesium silicate and magnesium aluminate; or organic compounds including primary, secondary, tertiary amines, cyclic amines, N,N'-dibenzylethylenediamine, diethanolamine, ethylenediamine, meglumine, monosodium glutamate, polacrilline sodium and sodium alginate.

9. The pharmaceutical tablet composition prepared according to claim 2 **characterized in that** the pharmaceutical tablet composition comprises a photoprotectant as a stabilizing agent selected from metal oxides including titanium oxide, ferric oxide and zinc oxide.

10. The pharmaceutical tablet composition prepared according to claim 2 **characterized in that** the pharmaceutically acceptable surfactant as an excipient is selected from polyoxyethylene-sorbitan-fatty acid esters (polysorbates), sodium lauryl sulfate, sodium stearyl fumarate, polyoxyethylene alkyl ethers, sorbitan fatty acid esters, polyethylene glycols, polyoxyethylene castor oil derivatives, docusate sodium, quaternary ammonium compounds, aminoacids including L-leucine, sugar esters of fatty acids and glycerides of fatty acids.

11. The pharmaceutical tablet composition according to claim 10, **characterized in that** the surfactant is present in an amount within the range of preferably 0 to 10% by weight, more preferably 0.1 to 5% by weight.

12. The pharmaceutical tablet composition according to any one of claims 2, 3, 5-11, **characterized in that** the pharmaceutical tablet composition preferably comprises the components given below.
desloratadine in an amount of from about 0.1 to 20% by weight;
montelukast sodium in an amount of from about 0.1 to 20% by weight;
mannitol in an amount of from about 0 to 95% by weight;
red iron oxide in an amount up to 1% by weight;
povidone in an amount of from about 0 to 10% by weight;
crospovidone in an amount of from about 0 to 10% by weight;
HPC in an amount of from about 0 to 10% by weight;
colloidal silicon dioxide in an amount up to 1% by weight;
L-leucine in an amount of from about 0 to 10% by weight;
calcium hydrogen phosphate dihydrate in an amount of from about 0 to 95% by weight;
microcrystalline cellulose in an amount of from about 0 to 95% by weight; corn starch in an amount of from about 0 to 10% by weight;
talk in an amount of from about 0 to 10% by weight;
citric acid anhydrous in an amount of from about 0.01 to 10% by weight; and sodium stearyl fumarate in an amount of from about 0 to 10% by weight.

13. The pharmaceutical tablet composition according to any one of claims 2, 3, and 5-11, **characterized in that** the pharmaceutical tablet composition preferably comprises the components given below.
desloratadine in an amount of from about 1 to 10% by weight;
montelukast sodium in an amount of from about 1 to 10% by weight;
mannitol in an amount of from about 5 to 85% by weight;
red iron oxide in an amount up to 1% by weight;
povidone in an amount of from about 0.1 to 5% by weight;
crospovidone in an amount of from about 0.1 to 5% by weight;
HPC in an amount of from about 0.1 to 5% by weight;
colloidal silicon dioxide in an amount up to 1% by weight;
L-leucine in an amount of from about 0.1 to 5% by weight;
calcium hydrogen phosphate dihydrate in an amount of from about 5 to 85% by weight;
microcrystalline cellulose in an amount of from about 5 to 85% by weight; corn starch in an amount of from about 0.1 to 5% by weight;
talk in an amount of from about 0.1 to 5% by weight;
citric acid anhydrous in an amount of from about 0.1 to 5% by weight; and sodium stearyl fumarate in an amount of from about 0.1 to 5% by weight.

## Patentansprüche

1. Ein Verfahren zur Herstellung einer pharmazeutischen Tablette, welcheeine therapeutisch wirksame Menge an Desloratadin oder ein davon abgeleitetes, pharmazeutisch verträglichesSalz, Solvat,Polymorph oder Hydrat sowie eine therapeutisch wirksame Menge an Montelukast oder ein in diesem Desloratadin gekennzeichneten,pharmazeutisch zulässigen Salzoder eindavon abgeleitetes, pharmazeutisch zulässiges Salz, Solvat, Polymorph oder Hydratund Montelukast oder eindavon abgeleitetes, pharmazeutisch verträglichesSalz beinhaltet undin einer Reihe vonHerstellungsschritten vorformuliert werden. Diese Schritte umfassen:
a. Die Herstellung der Vorformulierung von Desloratadin durch das Mischen von Desloratadin oder einem davon abgeleiteten, pharmazeutisch verträglichenSalz, Solvat, Polymorph oder Hydrat und mindestens einem pharmazeutisch verträglichenstabilisator, der aus der Gruppe Antioxidanten, Chelatbildner,Alkalisierungsmittel und Lichtschutzmittelausgewählt wurde, sowiegegebenenfalls pharmazeutisch verträgliche Hilfsstoffe;
b. Die Herstellung der Vorformulierung von Montekulast durch das Mischen von Montekulast oder einempharmazeutisch verträglichen, davon abgeleitetenSalz und mindestens einem pharmazeutisch verträglichen Stabilisator, der aus der Gruppe Antioxidanten, Chelatbildner, Alkalisierungsmittel und Lichtschutzmittelausgewählt wurde, sowie gegebenenfalls pharmazeutisch verträglichenHilfsstoffe;
c. die in denvorhergehendenSchritten hergestellten Vorformulierungen werdengesondert in dieTablettenpresse gegeben;
d. gegebenenfalls das Beschichten der in Schritt c) hergestellten Tabletten.

2. Eine pharmazeutische Tablette, die gemäßPatentanspruch1 hergestellt wurde.

3. Die pharmazeutische Tablette, die gemäß Patentanspruch 2 hergestellt wurde, ist durch die Zusammensetzung der Tablette gekennzeichnet. Diese beinhaltet in Bezug auf das Gewicht des Tablettenkerns:
- Desloratadin oder ein davon abgeleitetes, pharmazeutisch verträgliches Salz, Solvat, Derivat, Polymorph, Hydrat oder Enantiomerzu einer Menge von ungefähr 0,1 bis 20 Gewichtsprozent;
- Montekulast oder eindavon abgeleitetes, pharmazeutisch verträgliches Salzzu einer Menge von ungefähr 0,1 bis 20 Gewichtsprozent;
- mindestens einen pharmazeutischverträglichen Stabilisator für jede aktive Substanz zu einer Menge von ungefähr 10 Gewichtsprozent; und
- gegebenenfalls ein odermehrere pharmazeutisch verträgliche Hilfsstoffe, dieausdenZusatzstoffen wie Verdünnungsmitteln, Bindemitteln, Sprengmitteln, Schmiermitteln, GleitmittelnundTensidenausgewählt werden.

4. Die pharmazeutische Tablette mit einer inPatentanspruch2 beschriebenen Zusammensetzung für die Anwendung bei allergischen und entzündlichen Erkrankungen der Haut oder den oberen und unteren Atemwegen wie saisonal bedingteallergische Rhinitis, ganzjährigeallergischeRhinitis, allergischeSinusitis, Neurodermitis, Nesselfieber, allergisches Asthma, Aspirin-induziertesAsthma undbelastungsinduziertes Asthma.

5. Die pharmazeutische Tablette, die gemäß Patentanspruch 2 hergestellt wurde, ist durch die Zusammensetzung der Tablette gekennzeichnet und beinhaltet ein Natriumsalz von Montelukast als pharmazeutisch verträgliches Salz von Montekulast.

6. Die pharmazeutische Tablette, die gemäß Patentanspruch 2 hergestellt wurde, ist durch die Zusammensetzung der Tablette gekennzeichnet und beinhaltet ein Antioxidationsmittel als Stabilisierungsmittel, das aus Butylhydroxyanisol (BHA), Natriumascorbat, Butylhydroxytoluol (BHT), Natriumsulfit, Gallaten, Tocopherol, Zitronensäure, Apfelsäure, Ascorbinsäure, Acetylcystein, Fumarsäure, Lecithin, Ascorbylpalmitat und Ethylendiamin-tetraessigsäure beinhaltet.

7. Die pharmazeutische Tablette, die gemäß Patentanspruch 2 hergestellt wurde, ist durch die Zusammensetzung der Tablette gekennzeichnet und einen Chelatbildner als Stabilisierungsmittelbeinhaltet, der aus Ethylendiamintetraessigsäure, Zitronensäure, Natriumcitrat, Kaliumcitrat oder Kombinationendarausausgewählt wird.

8. Die pharmazeutische Tablette, die gemäß Patentanspruch 2 hergestellt wurde, ist durch die Zusammensetzung der Tablette gekennzeichnet undenthältals Stabilisator einAlkalisierungsmittel, das aus Alkalisalzen einschließlich Natriumcarbonat, Natriumhydroxid, Natriumsilikat, Dinatriumhydrogenorthophosphat und Natriumaluminatausgewählt wird; ErdalkalisalzeneinschließlichKalziumcarbonat, Kalziumhydroxid, Kalziumhydrogenphosphat, Tricalciumphosphat, Kalziumsulfat, Kalziumacetat,Kalziumglukonat, Kalziumglycerophosphat, Magnesiumcarbonat, Magnesiumhydroxid, Magnesiumsulfat, Magnesiumacetat, Magnesiumsilikat-und Magnesiumaluminat, oder organischeVerbindungen, einschließlichprimären, sekundären, tertiärenAminen,cyclischen Aminen, N,N'-Dibenzylethylendiamin, Diethanolamin, Ethylendiamin,Meglumin, Mononatriumglutamat, Polacrillin-Natrium undNatriumalginat enthält.

9. Die pharmazeutische Tablette, die gemäß Patentanspruch 2 hergestellt wurde, ist durch die Zusammensetzung der Tablette gekennzeichnet und beinhaltet ein Lichtschutzmittel als Stabilisator,dasaus MetalloxidenwieTitandioxid,Eisenoxidund Zinkoxid ausgewählt wird.

10. Die pharmazeutische Tablette, die gemäß Patentanspruch 2 hergestellt wurde, ist **dadurch gekennzeichnet, dass** sie einen pharmazeutisch verträgliches Tensid als Hilfsstoff enthält, der aus Polyoxyethylen - Sorbitan - Fettsäureester (Polysorbate), Natriumlaurylsulfat, Natriumstearylfumarat, Polyoxyethylenalkylether, Sorbitan-Fettsäureestern, Polyethylenglykolen, Polyoxyethylen - Rizinusöl - Derivaten, Docusatnatrium, quartären Ammoniumverbindungen, Aminosäuren wie L-Leucin,
Zuckerestervon Fettsäuren und Glyceridenvon Fettsäurenausgewählt wird.

11. Die pharmazeutische Tablette, die gemäß Patentanspruch 10 hergestellt wurde, ist **dadurch gekennzeichnet, dass** Tensid zu einer Menge von vorzugsweise 0 bis 10 Gewichtsprozent vorliegtundam besten zwischen 0,1 und 5 Gewichtsprozent.

12. Die pharmazeutische Tablette, die gemäß den Patentansprüchen 2, 3, 5-11hergestellt worden ist, ist **dadurch gekennzeichnet, dass** sie vorzugsweise eine der unten angegebenen Komponenten enthält.
Desloratadin in einer Menge von ungefähr 0,1 bis 20 Gewichtsprozent;
Montelukast-Natrium in einer Menge von ungefähr 0,1 bis 20 Gewichtsprozenten;
Mannit in einer Menge von ungefähr 0 bis 95 Gewichtsprozent;
rotes Eisenoxid in einer Menge bis zu 1 Gewichtsprozent;
Povidon in einer Menge von ungefähr 0 bis 10 Gewichtsprozent;
Crospovidon in einer Menge von ungefähr 0 bis 10 Gewichtsprozent;
HPC in einer Menge von ungefähr 0 bis 10 Gewichtsprozent;
kolloidales Siliciumdioxid in einer Menge bis zu 1 Gewichtsprozent;
L-Leucin in einer Menge von ungefähr 0 bis 10 Gewichtsprozent;
Calciumhydrogenphosphat-Dihydrat in einer Menge von ungefähr 0 bis 95 Gewichtsprozent;
mikrokristalline Cellulose in einer Menge von ungefähr 0 bis 95 Gewichtsprozent;
Maisstärke in einer Menge von ungefähr 0 bis 10 Gewichtsprozent;
Talk in einer Menge von ungefähr 0 bis 10 Gewichtsprozent;
Zitronensäure in einer Menge von ungefähr 0,01 bis 10 Gewichtsprozent, und Natriumstearylfumarat in einer Menge von ungefähr 0 bis 10 Gewichtsprozent.

13. Die pharmazeutische Tablette, die gemäß den Patentansprüchen 2, 3, 5-11hergestellt worden ist, ist **dadurch gekennzeichnet, dass** sie vorzugsweise eine der unten angegebenenKomponenten enthält.
Desloratadin in einer Menge von ungefähr 1 bis 10 Gewichtsprozent;
Montelukast-Natrium in einer Menge von ungefähr 1 bis 10 Gewichtsprozent;
Mannit in einer Menge von ungefähr 5 bis 85 Gewichtsprozent ;
rotes Eisenoxid in einer Menge bis zu 1 Gewichtsprozent;
Povidon in einer Menge von ungefähr 0,1 bis 5 Gewichtsprozent;
Crospovidon in einer Menge von ungefähr 0,1 bis 5 Gewichtsprozent;
HPC in einer Menge von ungefähr 0,1 bis 5 Gewichtsprozent;
kolloidales Siliciumdioxid in einer Menge bis zu 1 Gewichtsprozent;
L- Leucin in einer Menge von ungefähr 0,1 bis 5 Gewichtsprozent;
Calciumhydrogenphosphat-Dihydrat in einer Menge von ungefähr 5 bis 85 Gewichtsprozent ;
mikrokristalline Cellulose in einer Menge von ungefähr 5 bis 85 Gewichtsprozent ;
Maisstärke in einer Menge von ungefähr 0,1 bis 5 Gewichtsprozent;
sprechen in einer Menge von ungefähr 0,1 bis 5 Gewichtsprozent;
Zitronensäure in einer Menge von ungefähr 0,1 bis 5 Gewichtsprozent , und
Natriumstearylfumarat in einer Menge von ungefähr 0,1 bis 5 Gewichtsprozent.

## Revendications

1. Procédé pour la préparation d'une composition de comprimé pharmaceutique comprenant une quantité thérapeutiquement efficace de desloratadine ou un de ses sels, de ses solvates, de ses polymorphes ou de ses hydrates pharmaceutiquement acceptable et une quantité thérapeutiquement efficace de Montelukast ou un de ses sels pharmaceutiquement acceptable, caractérisé en ce quele desloratadine ou un de ses sels, de ses solvates, de ses polymorphes pharmaceutiquement acceptables ou l'un de ses hydrates et le montelukast ou un de ses sels pharmaceutiquement acceptables sont préformulés dans une série d'étapes d'une série de processus de fabrication comprenant :
a. La préparation de la préformulation de desloratadine à travers le mélange de cet antihistaminique ou un de ses sels, solvates, polymorphes ou hydrates pharmaceutiquement acceptables avec au moins un agent stabilisant pharmaceutiquement acceptable choisi dans le groupe des antioxydants, agents chélatants et alcalinisants, photoprotectants et, en option, d'autres excipients pharmaceutiquement acceptables,
b. La préparation de la préformulation du montelukast à travers un mélange de montelukast ou un de ses sels pharmaceutiquement acceptables et au moins un agent stabilisant pharmaceutiquement acceptable choisi dans le groupe des antioxydants, agents chélatants et alcalinisants, photoprotectants et, en option, d'autres excipients pharmaceutiquement acceptables,
c. Les préformulations obtenues séparément des étapes précédentes sont compressés dans la machine à réalisation de comprimés.
d. En option, enrobage des comprimés obtenus à l'étape c).

2. Une composition de comprimé pharmaceutiquepréparée selon la revendication 1.

3. La composition pharmaceutique du comprimé préparée selon la revendication 2,**caractérisé en ce que** la composition pharmaceutique comprend, par rapport au poids du comprimé de base,
- le desloratadine ou un de ses sels, solvates, dérivés, polymorphes, hydrates ou énantiomères pharmaceutiquement acceptables en une quantité allant d'environ 0,1 %à20%,
- le montelukast ou un de ses sels pharmaceutiquement acceptable en une quantité allant d'environ 0,1 % à 20 %,
- pour chacune des substances actives,au minimum un agent stabilisant pharmaceutiquement acceptable en une quantité pouvant atteindre jusqu'à 10 %, et
- en option, un ou plusieurs excipients pharmaceutiquement acceptables choisis parmi les additifs tel que les agents diluants, liants, désintégrants, lubrifiants,glissants, tensioactifs.

4. La composition de comprimé pharmaceutique préparée selon la revendication 2 est utilisée pour le traitement des affections allergiques et inflammatoires de la peau ou des voies respiratoires supérieures et inférieures telles que les rhinites allergiques saisonnières, les rhinites allergiques perannuelles, les sinusites allergiques, les dermatites atopiques, les urticaires, les asthmes allergiques, l'asthme provoqué par l'aspirine, l'asthme provoqué par l'exercice physiques.

5. La composition de comprimé pharmaceutique préparée conformément à la revendication 2 est **caractérisée en ce qu'**elle contient du sel de sodium de montelukast ajouté comme sel de montelukast pharmaceutiquement acceptable.

6. La composition de comprimé pharmaceutique préparée conformément à la revendication 2 est **caractérisée en ce qu'**elle contient un antioxydant qui joue un rôle d'agent stabilisant sélectionné à partir du butylhydroxyanisole (BHA), de l'ascorbate de sodium, de l'hydroxytoluène butylé (BHT), du sulfite de sodium, de la galatte, du tocophérol, de l'acide citrique, de l'acide malique, de l'acide ascorbique, de l'acétylcystéine, de l'acide fumarique, de la lécithine, de la palmitate d'ascorbyle, ainsi que de l'éthylène diamine tétra acétate.

7. La composition de comprimé pharmaceutique préparée conformément à la revendication 2 est **caractérisée en ce qu'**elle contient une substance chélatante qui joue le rôle d'agent stabilisant sélectionné à partir de l'éthylènedinitrilotétraacétate de sodium, de l'acide édétique, de l'acide citrique, du citrate de sodium, du citrate de potassium ou de leurs combinaisons.

8. La composition de comprimé pharmaceutique préparée conformément à la revendication 2 est **caractérisée en ce qu'**elle contient une substance alcalinisante comme agent stabilisant sélectionné à partir des sels de métaux alcalins dont du carbonate de sodium, de l'hydroxyde de sodium, du silicate de sodium, de l'orthophosphate bisodique, de l'aluminate de soude, des sels de métaux alcalinoterreux dont du carbonate de calcium, de l'hydroxyde de calcium, du phosphate de calcium dibasique, du phosphate de calcium tribasique, du sulfate de calcium, de l'acétate de calcium, du gluconate de calcium, du glycérophosphate de calcium, du carbonate de magnésium, de l'hydroxyde de magnésium, du sulfate de magnésium, de l'acétate de magnésium, du silicate de magnésium ainsi que de l'aluminate de magnésium incluant ou des amines primaires, secondaires, tertiaires, et cycliques, de la N,N'-dibenzyléthylènediamine, de la diéthanolamine, de l'éthylènediamine, de la méglumine, du glutamate monosodique, de la polacriline sodique, et de l'alginate de sodium.

9. La composition de comprimé pharmaceutique préparée conformément à la revendication 2 est **caractérisée en ce qu'**elle contient un photoprotectant qui joue le rôle d'agent stabilisant sélectionné à partir des oxydes métalliques incluant l'oxyde de titane, l'oxyde ferrique et l'oxyde de zinc.

10. La composition de comprimé pharmaceutique préparée conformément à la revendication 2 est **caractérisée en ce que** le tensioactif pharmaceutiquement acceptable comme excipient est sélectionné à partir des esters d'acide gras du polyoxyéthylènesorbitane (polysorbates), dulauryl sulfate de sodium, du stéarylfumarate de sodium, des éthers alkyliques de polyoxyéthylène, des esthers d'acide gras du sorbitane, des polyéthylènes glycols, des dérivés d'huile de ricin de polyoxyéthylène, du docusate sodique, des composés d'ammonium quaternaire, des amino-acides dont la L-leucine, les esters de sucre d'acides gras, ainsi que les glycérides d'acides gras.

11. La composition de comprimé pharmaceutique préparée conformément à la revendication 10 est **caractérisée en ce que** le tensioactif est présent en une quantité comprise dans l'intervalle de 0 % et 10 % en poids, et de préférence de 0,1 % à 5 % en poids.

12. La composition de comprimé pharmaceutique préparée conformément aux revendications 2, 3, 5-11, est **caractérisée en ce qu'**elle contient de préférence les composants mentionnés ci-dessous :
Une quantité en poids de desloratadine comprise environ entre 0,1 % et 20 % ;
une quantité en poids de montélukast sodique comprise environ entre 0,1 % et 20%;
une quantité en poids de mannitol comprise environ entre 0 % et 95 % ;
une quantité en poids d'oxyde de fer rouge allant jusqu'à 1 % ;
une quantité en poids de povidone comprise environ entre 0 % et 10 % ;
une quantité en poids de crospovidone comprise environ entre 0 % et 10 % ;
une quantité en poids de HPC comprise environ entre 0 % et 10 % ;
une quantité en poids de dioxyde de silicium colloïdal allant jusqu'à 1 % ;
une quantité en poids de L-Leucine comprise environ entre 0 % et 10 % ;
une quantité en poids de dihydrate de phosphate d'hydrogène de calcium comprise environ entre 0 % et 95 % ;
une quantité en poids de cellulose microcristalline comprise environ entre 0 % et 95 % ;
une quantité en poids d'amidon de maïs comprise environ entre 0 % et 10 % ;
une quantité en poids de talc comprise environ entre 0 % et 10 % ;
une quantité en poids d'acide citrique anhydre comprise environ entre 0,01 % et 10 % ; et
une quantité en poids de stéarylfumarate de sodium comprise environ entre 0 % et 10 %.

13. La composition de comprimé pharmaceutique préparée conformément aux revendications 2, 3, 5-11, est **caractérisée en ce qu'**elle contient de préférence les composants mentionnés ci-dessous :
une quantité en poids de desloratadine comprise environ entre 1 % et 10 % ;
une quantité en poids de montélukast sodique comprise environ entre 1 % et 10% ;
une quantité en poids de mannitol comprise environ entre 5 % et 85 % ;
une quantité en poids d'oxyde de fer rouge allant jusqu'à 1 % ;
une quantité en poids de povidone comprise environ entre 0,1 % et 5 % ;
une quantité en poids de crospovidone comprise environ entre 0,1 % et 5 % ;
une quantité en poids de HPC comprise environ entre 0,1 % et 5 % ;
une quantité en poids de dioxyde de silicium colloïdal allant jusqu'à 1 % ;
une quantité en poids de L-Leucine comprise environ entre 0,1 % et 5 % ;
une quantité en poids de dihydrate de phosphate d'hydrogène de calcium comprise environ entre 5 % et 85 % ;
une quantité en poids de cellulose microcristalline comprise environ entre 5 % et 85 % ;
une quantité en poids d'amidon de maïs comprise environ entre 0,1 % et 5 % ;
une quantité en poids de talc comprise environ entre 0,1 % et 5 % ;
une quantité en poids d'acide citrique anhydre comprise environ entre 0,1 % et 5 % ; et
une quantité en poids de stéarylfumarate de sodium comprise environ entre 0,1 % et 5 %.
